## Europäisches Patentamt
### European Patent Office
### Office européen des brevets

(11) Publication number: **0 189 755**
**B1**

# EUROPEAN PATENT SPECIFICATION

(45) Date of publication of patent specification: **03.01.90**

(51) Int. Cl.⁵: **A 61 K 31/70**

(21) Application number: **86100150.1**

(22) Date of filing: **08.01.86**

(54) **Pharmaceutical composition.**

(30) Priority: **30.01.85 EP 85100939**

(43) Date of publication of application:
**06.08.86 Bulletin 86/32**

(45) Publication of the grant of the patent:
**03.01.90 Bulletin 90/01**

(84) Designated Contracting States:
**CH DE FR GB IT LI**

(56) References cited:
**EP-A-0 048 022**

**CHEMICAL ABSTRACTS, vol. 100, no. 17, 23rd April 1984, page 35, no. 132250z, Columbus, Ohio, US; M. IIGO et al.: "Potentiation of antitumor activity of 5-fluoro-2-deoxyuridine by guanosine 5′-monophosphate", & J. PHARMACOBIO-DYN. 1984, 7(1), 67-9**

(73) Proprietor: **F. HOFFMANN-LA ROCHE AG**
**Postfach 3255**
**CH-4002 Basel (CH)**

(72) Inventor: **Iigo, Masaaki National Cancer Center Shukusha C-32 Tsukiji 5-1-1 Chuo-ku Tokyo (JP)**
Inventor: **Miwa, Masanori Kamakura Green Mansion 404, Ueki 436-1 Kamakura-shi Kanagawa-ken (JP)**
Inventor: **Nitta, Kazuo Irima-cho 1-24-16 Chofu-shi Tokyo (JP)**

(74) Representative: **Mahé, Jean et al Postfach 3255 Grenzacherstrasse 124 CH-4002 Basel (CH)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**Description**

This invention is concerned with a novel pharmaceutical composition. More particularly, this invention is concerned with a novel pharmaceutical composition, which contains, as the active substance, a mixture of (a) 5'-deoxy-5-fluorouridine, hereinafter referred to as 5'-DFUR, or a pharmaceutically acceptable salt thereof; and (b) a purine nucleoside or a purine nucleotide; still more particularly with such a composition when used as an antitumor agent.

It has been known that 5'-DFUR is useful as an antitumor agent and its therapeutic indices are several times higher than that of 5-fluorouracil, hereinafter referred to as 5-FU. It has now surprisingly been found that the antitumor activity of the mixture of the above mentioned compounds (a) and (b) is remarkably superior to that of 5'-DFUR, without any increase in toxicity.

In a preferred embodiment, the pharmaceutical composition provided by the present invention may contain the active substances in the weight ratio of the above mentioned compound (a) to compound (b) of 1:0.1 to 1:20, more preferably 1:1 to 1:10.

In the present invention, preferred examples of the pharmaceutically acceptable salts of 5'-DFUR are non-toxic salts such as those formed with acids selected from inorganic mineral acids and organic acids, such as hydrochloride, hydrobromide, phosphate, sulphate, nitrate, acetate, formate, maleate, fumarate, and benzoate; and preferred examples of pruine nucleosides and purine nucleotides are, for example, guanosine, adenosine, inosine, xanthosine, guanosine-5'-monophosphate, guanosine-5'-diphosphate, guanosine-5'-triphosphate, adenosine-5'-monophosphate, adenosine-5'-diphosphate, adenosine-5'-triphosphate, inosine-5'-monophosphate, inosine,-5'-diphosphate, inosine-5'-triphosphate, xanthosine-5'-monophosphate, xanthosine-5'-diphosphate and xanthosine-5'-triphosphate.

The pharmaceutical composition provided by the present invention can be useful for the treatment of tumors, such as breast cancer, stomach cancer, colorectal cancer, pancreatic cancer, head and neck cancer and lung cancer.

The pharmaceutical composition provided by the present invention may contain the combination of the aforementioned active substances in association with a compatible pharmaceutical carrier material. This carrier material can be an organic or inorganic inert carrier material suitable for enteral, percutaneous or parenteral administration, such as water, gelatine, gum arabic, lactose, starch, magnesium stearate, talc, vegetable oils, polyalkylene glycols and petroleum jelly. The pharmaceutical composition provided by the present invention can be administered orally, e.g. in the form of tablets, capsules, pills, powders, granulates, solutions, syrups, suspensions or elixirs. The administration can also be carried out parenterally, e.g. in the form of sterile solutions, suspensions or emulsions; or locally, e.g. in the form of solutions, suspensions, salves, powders or aerosols. The pharmaceutical composition can be sterilized and/or can contain further adjuvants such as preserving, stabilizing, setting, emulsifying agents, flavour-improving agents, salts for variation of the osmotic pressure or subtances acting as buffers. The pharmaceutical composition can be prepared in a conventional manner.

The dosage of the active substances depends on the route of administration, the age, weight and condition of the patient and the particular disease to be treated. However, a typical dosage for adults is in the range of 100 to 6,000 mg/body/day in the case of oral, rectal or parenteral administration. Rectal administration and intravenous injection are preferred routes of administration of the pharmaceutical composition according to the present invention.

The enhanced antitumor activity of the pharmaceutical composition in accordance with the present invention is evident from the tests described hereinafter.

(1) *Antitumor effects of combination of 5'-DFUR with guanosine 5'-monophosphate (hereinafter referred to as GMP)*

$BDF_1$ male mice weighing 21 to 23 g were used as experimental animals. P388 leukemia cells were implanted intraperitoneally into the mice. Seven days after the implantation, ascites were collected and diluted with saline to prepare the suspension containing $10^6$ P388 leukemia cells in 0.05 ml. The mice were intraperitoneally implanted on day 0 with $10^6$ P388 cells. Each group contained 6 mice. 5'-DFUR and GMP were dissolved in saline, alone or combined with each other. The test compounds were administered intraperitoneally from day 1 to day 5. The antitumor effects were evaluated by the increase in life span over control (ILS) calculated by the following formula:

$$ILS = T/C\% - 100$$

T: Mean survival time (days) in the test drug groups
C: Mean survival time (days) in the control group administered intraperitoneally with saline alone.

The safety of the drug was evaluated by the therapeutic ratio: the dose $ILS_{max}$/the dose $ILS_{30}$.

As can be seen from Table 1, when the dose of 5'-DFUR was in the range from 2 to 250 mg/kg/day, the higher the dose of 5'-DFUR was, the longer the survival time was, both in the group receiving 5'-DFUR alone and in the group receiving 5'-DFUR combined with GMP at 100 mg/kg/day. The $ILS_{max}$ was 48% in the 5'-DFUR alone group, while 107% in the 5'-DFUR-GMP combination group.

EP 0 189 755 B1

## TABLE 1
### Effects of GMP (100 mg/kg/day) on the antitumor activity of 5′-DFUR against P388 leukemia

| Dose of 5′-DFUR (mg/kg/day i.p.) | ILS (%) | |
|---|---|---|
| | 5′-DFUR alone | 5′-DUR + GMP |
| 2 | 0 | 31 |
| 4 | 1 | 56 |
| 8 | 8 | 61 |
| 16 | 15 | 73 |
| 32 | 21 | 76 |
| 63 | 21 | 82 |
| 125 | 31 | 101 |
| 250 | 48 | 107 |
| 500 | 24 | 71 |

Note: Mean survival time in the control group: 10.7 days.

The therapeutic ratio was 2 (250 mg/125 mg) in the 5′-DFUR alone group, while 125 (250 mg/2 mg) in the 5′-DFUR-GMP combination group. The therapeutic ratio in the 5′-DFUR-GMP combination group was 63 times as high as in the 5′-DFUR alone group.

Moreover, as the dose of GMP was increased, the ILS got high accordingly (see Table 2) without increasing any toxic effects. With GMP alone, there was no influence observed on ILS (see Table 3).

## TABLE 2
### Dose examination of GMP on the antitumor activity of 5′-DFUR against P388 leukemia

| Dose of 5′-DFUR (mg/kg/day i.p.) | Dose of GMP (mg/kg/day i.p.) | ILS (%) |
|---|---|---|
| 250 | 0 | 48 |
| 250 | 100 | 107 |
| 250 | 200 | 112 |
| 250 | 300 | 120 |
| 250 | 500 | 125 |
| 250 | 1000 | 130 |

## TABLE 3
### The antitumor activity of GMP against P388 leukemia

| Dose of 5′-DFUR (mg/kg/day i.p.) | Dose of GMP (mg/kg/day i.p.) | ILS (%) |
|---|---|---|
| 0 | 100 | −3 |
| 0 | 300 | 5 |
| 0 | 500 | 8 |
| 0 | 1000 | 1 |

3

The antitumor effects of the combination of 5'-DFUR with GMP have been compared with those of the combination of 5-FU with GMP (Table 4). The therapeutic ratio was 2 with 5'-DFUR alone and 125 with the combination of 5'-DFUR with GMP, 63 times as high as with 5'-DFUR alone, while it was 26.6 with 5-FU alone and 74.0 with the combination of 5-FU with GMP, 2.8 times as high as with 5-FU alone. The therapeutic ratio of the combination of 5'-DFUR with GMP was higher than that of the combination of 5-FU with GMP. Accordingly, the combination of 5'-DFUR with GMP is considered very significant for cancer chemotherapy.

TABLE 4

Comparison of the combined effect of GMP (100 mg/kg/day) and 5-FU or 5'-DFUR

|  | 5'-DFUR alone | 5'-DFUR + GMP | 5-FU alone | 5-FU + GMP |
|---|---|---|---|---|
| Therapeutic ratio (dose $ILS_{max}$/dose $ILS_{30}$) | 2 (250/125) | 125 (250/2) | 26.6 (20/0.75) | 74.0 (20/0.27) |

(2) *Antitumor effects of combination of 5'-DFUR with purine nucleotides*

In a manner analogous to that described in (1) above, except that instead of GMP, adenosine-5'-monophosphate or inosine-5'-monophosphate (hereinafter respectively referred to as AMP and IMP) was used, the antitumor effects were tested. The results are given in Table 5.

TABLE 5

| Compound Dose (mg/kg/day) | Change of body weight on days 6 (g) | Survival days (means ± SD) | T/C (%) |
|---|---|---|---|
| 5'-DFUR (250) | − 2.5 | 13.03 ± 0.8 | 139 |
| 5'-DFUR + GMP (250 + 100) | − 2.3 | 16.5 ± 1.4 | 172 |
| 5'-DFUR + AMP (250 + 100) | − 3.4 | 16.7 ± 0.5 | 174 |
| 5'-DFUR + IMP (250 + 100) | + 2.4 | 16.0 ± 1.8 | 167 |
| GMP (100) | + 1.5 | 8.8 ± 0.8 | 92 |
| AMP (100) | + 0.9 | 9.5 ± 1.9 | 99 |
| IMP (100) | + 1.5 | 9.7 ± 1.8 | 101 |
| Saline | + 1.1 | 9.6 ± 1.6 | — |

(3) *Antitumor effects of combination of 5'-deoxy-5-fluorocytidine (hereinafter referred to as 5'-DFCR) with GMP*

In a manner analogous to that described in (1) above except that 5'-DFCR was used instead of 5'-DFUR, the antitumor effects thereof was tested as a referential example. No enhancement of antitumor effects was observed with the combination of 5'-DFCR and GMP, as can be seen from Table 6.

EP 0 189 755 B1

TABLE 6

| Compound Dose (mg/kg/day) | GMP (100 mg/kg/day) | | | | | |
|---|---|---|---|---|---|---|
| | without | | | with | | |
| | Change of body weight on days 6 (g) | Survival days (means ± SD) | T/C (%) | Change of body weight on days 6 (g) | Survival days (mean ± SD) | T/C (%) |
| 5'—DFCR | | | | | | |
| 28 | +0.2 | 10.7 ± 1.5 | 111 | +0.4 | 10.0 ± 0.6 | 104 |
| 55 | +1.0 | 10.0 ± 1.7 | 104 | +0.9 | 11.2 ± 1.6 | 117 |
| 110 | +0.6 | 12.0 ± 0.5 | 107 | ±0.0 | 11.0 ± 0.6 | 115 |
| 220 | −0.8 | 12.0 ± 1.7 | 125 | −0.3 | 12.0 ± 0.0 | 125 |
| Saline | +1.1 | 9.6 ± 1.6 | — | +1.5 | 8.8 ± 0.8 | 92 |

Acute toxicity

Groups of 5 weeks old male ICR mice were treated intraperitoneally with 5'—DFUR and GMP at the dosage given in the following Table 7. Each mice group contained 5 mice. Two weeks after the number of dead mice were counted. The quotient number of dead mice/number of tested mice is given in Table 7.

TABLE 7

| 5'—DFUR (mg/kg) \ GMP (mg/kg) | 0 | 2000 | 4000 |
|---|---|---|---|
| 0 | not done | 0/5 | 1/5 |
| 2000 | 0/5 | not done | 6/6 |
| 4000 | 0/5 | 0/5 | 5/5 |

The followings are examples of a typical dosage form:

1. Injection in a vial

| | |
|---|---|
| 5'—DFUR | 500 mg |
| GMP | 2,000 mg |
| 5N NaOH | 370 µl |
| Distilled water | 5 ml |

2. Injection in a vial

| | |
|---|---|
| 5'—DFUR | 1,000 mg |
| GMP | 2,000 mg |
| 5N NaOH | 446 µl |
| Distilled water | 10 ml |

**Claims**

1. A pharmaceutical composition containing as the active substance, a mixture of 5'-deoxy-5-fluorouridine or a pharmaceutically acceptable salt thereof; and a purine nucleoside or a purine nucleotide.

5

2. A pharmaceutical composition according to claim 1 as antitumor agent.

3. A pharmaceutical composition according to claim 1 or 2, wherein the weight ratio of 5'-deoxy-5-fluorouridine or the pharmaceutically acceptable salt thereof to the purine nucleoside or the purine nucleotide is 1:0.1 to 1:20, particularly 1:1 to 1:10.

4. A pharmaceutical composition according to claim 1, 2 or 3, which contains a mixture of 5'-deoxy-5-fluorouridine and guanosine-5'-monophosphate.

5. The use of a mixture of 5'-deoxy-5-fluorouridine or a pharmaceutically acceptable salt thereof; and a purine nucleoside or a purine nucleotide, for the manufacture of a pharmaceutical composition according to any one of claims 1—4.

## Patentansprüche

1. Ein pharmazeutisches Präparat, enthaltend als Wirkstoff ein Gemisch des 5'-Deoxy-5-fluoruridins oder eines pharmazeutisch akzeptblen Salzes davon; und eines Purinnuclosids oder eines Purinnucleotids.

2. Ein pharmazeutisches Präparat gemäss Anspruch 1 als Antitumormittel.

3. Ein pharmazeutisches Präparat gemäss Anspruch 1 oder 2, worin das Gewichtsverhältnis des 5'-Deoxy-5-fluoruridins oder eines pharmazeutisch akzeptablen Salzes davon zum Purinnucleosid oder Purinnucleotid von 1:0,1 bis 1:20, insbesondere von 1:1 bis 1:10 ist.

4. Ein pharmazeutisches Präparat gemäss Anspruch 1, 2 oder 3, enthaltend ein Gemisch von 5'-Deoxy-5-fluoruiridin und Guanosin-5'-monophosphat.

5. Die Verwendung eines Gemisches des 5'-Deoxy-5-fluoruridins oder eines pharmazeutisch akzeptablen Salzes davon; und eines Purinnucleosides oder eines Purinnucleotids für die Herstellung eines pharmazeutischen Präparats gemäss einem der Ansprüche 1—4.

## Revendications

1. Composition pharmaceutique contenant comme substance active un mélange de 5'-désoxy-5-fluorouridine ou d'un de ses sels pharmaceutiquement acceptables; et d'un nucléoside purique ou nucléotide purique.

2. Composition pharmaceutique selon la revendication 1 comme agent antitumoral.

3. Composition pharmaceutique selon la revendication 1 ou 2, dans laquelle le rapport pondéral de la 5'-désoxy-5-fluoroudine ou de son sel pharmaceutiquement acceptable au nucléoside purique ou au nucléotide purique est de 1:0 à 1:20, en particulier de 1:1 à 1:10.

4. Composition pharmaceutique selon la revendication 1, 2 ou 3 qui contient un mélange de 5'-désoxy-5-fluorouridine et de guanosine-5'-monophosphate.

5. Application d'un mélange de 5'-désoxy-5-fluorouridine ou d'un de ses sels pharmaceutiquement acceptables; et d'un nucléoside purique ou d'un nucléotide purique, à la préparation d'un composition pharmaceutique selon l'une quelconque des revendications 1—4.